# EUROPEAN PATENT SPECIFICATION

(11) **EP 1 919 304 B1**
(45) Date of publication and mention of the grant of the patent: **25.05.2011**
(21) Application number: 06777068.5
(22) Date of filing: 25.08.2006
(51) Int. Cl.: A23L 1/305, A23L 1/30, A61K 31/198, A61K 31/202, A23K 1/18, A23K 1/16, A61P 9/10, A61P 25/00

(54) **COMPOSITIONS AND METHODS FOR IMPROVING FUNCTIONAL VASCULAR INTEGRITY, CELLULAR SURVIVAL AND REDUCING APOPTOSIS AFTER AN ISCHEMIC EPISODE IN THE BRAIN**
ZUSAMMENSETZUNGEN UND VERFAHREN ZUR VERBESSERUNG DER FUNKTIONALEN GEFÄßINTEGRITÄT, DES ÜBERLEBENS DER ZELLEN UND DER REDUCTION DER APOPTOSE NACH EINEM ISCHÄMIEANFALL IM GEHIRN
COMPOSITIONS ET METHODES DESTINEES A AMELIORER L'INTEGRITE VASCULAIRE FONCTIONNELLE ET LA SURVIE CELLULAIRE ET A REDUIRE L'APOPTOSE APRES UN EPISODE ISCHEMIQUE DANS LE CERVEAU

(30) Priority: 26.08.2005 US 711547 P; 26.08.2005 US 711549 P
(43) Date of publication of application: 14.05.2008
(73) Proprietor: Nestec S.A., 1800 Vevey (CH)
(72) Inventor: PAN, Yuanlong, MO 63005 (US); LARSON, Brian, Dowling MI 49050 (US)
(74) Representative: Lock, Graham James
(86) International application number: PCT/EP2006/008354
(87) International publication number: WO 2007/022991

(56) References cited:
- EP-A- 1 665 940
- EP-A1- 0 699 437
- WO-A-2005/023021
- WO-A2-2006/032115
- DE-A1- 10 214 005
- US-A- 4 920 098
- US-A- 5 731 290
- US-A1- 2002 018 807
- US-A1- 2002 182 196

## Description

### FIELD OF THE INVENTION

The present invention relates to mammalian nutrition and effects thereof on the enhancing vascular integrity and protecting against cellular damage associated with ischemia or ischemic episode in the brain. In particular, the present invention utilizes combinations of nitric oxide releasing compounds, long chain polyunsaturated fatty acids and optionally other ingredients including antioxidants, anti-inflammatory agents, growth factors and B-vitamins, administered as part of a regular diet, to improve vascular integrity, promote cellular survival, and thereby reduce ischemic injury in the brain and/ or other organs, for example in the event of a brain ischemia.

### BACKGROUND OF THE INVENTION

Various publications, including patents, published applications, technical articles and scholarly articles are cited throughout the specification.

Hypoxic ischemic brain injury occurs as the result of diminished oxygen supply to the brain due to a transient or permanent reduction or stoppage of blood flow to the brain. Ischemia of the brain results in a stroke, with subsequent apoptosis and necrosis of brain tissue leading to an infarction. Similar to cardiovascular ischemia, brain ischemia can be caused by various factors such as blood clots, thrombosis, embolism, blockage by atherosclerotic plaques, or other obstructions in the vasculature. Hypercholesterolemia, hypertension, diabetes, and obesity, among other factors, have been identified as risk factors for ischemic strokes. Ischemic strokes are a leading cause of death of human beings worldwide, and also affect other animals, including companion animals.

Ischemic lesions are also known to contribute significantly to brain aging and dementia. Dementia is a major disease affecting the elder population. The incidence of dementia increases from about 0.3 to 1% in people aged 60 to 64 years to 50% in people older than 95 years with the prevalence doubling every 5 years (McDowell, I., Aging (Milano) 13: (2001) 143-162). In the Western populations, Alzheimer's disease (AD) is considered the major form of dementia, while vascular dementia (VD) appears to be the predominant form in Eastern populations (Fratiglioni, L. et al., Drugs Aging (1999) 15: 365-375; Ott, A., et al., The Rotterdam Study, BMJ (1995) 310: 970-973). Growing evidence indicate that vascular dementia and Alzheimer's disease often coexist in the population with dementia. For instance, Kalaria (Neurobiol. Aging (2000) 21: 321-330) reported that about 90% of patients with AD had cerebrovascular pathology, and about 30% of VD patients had AD pathology. The coexistence of cerebrovascular lesion and AD in demented elderly people was confirmed by Snowdon et al. (J. Am. Med. Soc. (1997) 277: 813-817). In addition, the hallmark of the pathological diagnosis of AD (amyloid deposition, and neurofibrillary tangles) was determined to exist in non-demented elderly people (Snowdon et al., 1997, *supra*). These data suggest that amyloid deposition, and neurofibrillary tangles are not enough to cause dementia in AD patients, highlighting the significant contribution of brain ischemic lesions to brain aging, and the development and progression of both VD and AD (Korczyn, A. J. Neurol. Sci. (2005) 230: 3-6). Therefore preventing ischemia-induced brain lesion and apoptosis of neurons in humans and animals may retard brain aging, reduce the risk of cognitive impairment, and retard the progression of dementia.

Ischemia can occur in any tissue, but is most commonly associated with brain, cardiac or kidney damage. As discussed above, ischemia in the brain can cause acute damage, e.g., stroke, or chronic impairments such as dementia and related conditions. In the heart, ischemic episodes can result in heart failure; likewise kidney failure may result from ischemic events in the kidney.

During ischemic stroke or episodes of the brain or any other tissue, biochemical reactions occur in the vasculature that may lead to edema, hemorrhagic transformation, and a further compromise in the affected tissue, for example neurologic. As such, treatment and protection of the vasculature has been identified as a potential avenue to explore in acute ischemic stroke in order to develop new therapies. Moreover, vascular protection has implications to reduce tissue damage that occurs as a result of an ischemic episode.

The vascular endothelium has been determined to serve various regulatory functions, including modulation of vascular tone, inflammation, and homeostasis by maintaining a non-adhesive, anti-thrombotic surface. (Boak, L., et al. Cur. Vasc. Pharmacol. (2004) 2:45-52). Vascular homeostasis is in part a function of the vascular smooth muscle contraction and relaxation, which is mediated by a variety of factors, including the free radical nitric oxide (NO), which is a strong relaxant of the vascular smooth muscle.

NO is produced by three different NO synthase (NOS) enzymes, neuronal NOS (nNOS), which is primarily localized in nervous tissue and generates NO for neurotransmission; inducible NOS (iNOS), which is found primarily in macrophages and may respond to proinflammatory mediators; and endothelial NOS (eNOS), which is produced by endothelial cells such as those found in the vascular endothelium. (Michel, T., et al. J Clin. Invest. (1997) 100: 2146-2152, Moncada, S., et al. Pharmacol. Rev. (1991) 43:109-142, and, Nathan, C. (1992) FASEB J 6: 3051-3064). NO released by eNOS plays a major role in vasodilation, smooth muscle proliferation, regulation of arterial blood pressure, and has anticoagulant and anti-inflammatory effects via inhibition of platelet and leukocyte adhesion and aggregation. (Gewaltig, M.T., et al. Cardiovasc. Res. (2002) 55:250-260, De Graaf, J.C. et al. Circulation (1992) 85:2284-2290, Freedman, J.E., et al. Circ. Res. (1999) 84:1416-1421, Furchgott, R.F., et al. FASEB J (1989) 3:2007-2018, Gaboury, J., et al. Am J Physiol. Heart. Circ. Physiol. (1993) 265: H862-H867, and, Kubes, P., et al. Proc. Natl. Acad. Sci. USA (1991) 88: 4651-4655). A decrease in NO production during prolonged ischemia has been associated with endothelial injury (Laude, L., et al. Braz. J Med. Biol. Res. (2001) 34:1-7).

The NOS enzymes oxidize L-arginine to citrulline, resulting in the formation of NO as a byproduct. Thus, it has been suggested that supplementation with NO precursors such as L-arginine, can improve vascular health, and may facilitate repair of vascular disease states. Indeed, dietary arginine supplementation has been shown to induce restoration of vasodilation and improve coronary circulation in animal models and human patients with hypercholesterolemia, as well as enhance overall endothelial function in patients with coronary artery disease. (Boak, L., *et al.* 2004). In addition, L-arginine supplementation has also been shown to increase NO levels and attenuate free O₂ radical-mediated myocardial injury in human patients. (Kiziltepe, U., et al. Int. J Cardiol. (2004) 97:93-100) . More recent studies have shown that arginine supplementation facilitates endothelial repair in the intestine of rats following induced intestinal ischemia-reperfusion. (Sukhotnik, I., et al., Pediatr. Surg. Int. (2005) 3:191-196). Thus, administration of NO precursors such as L-arginine have implications for endothelial cell activation and protection, especially with respect to vascular endothelial cells.

Fatty acids have also been demonstrated to modulate endothelial cell activation. Administration of the long chain polyunsaturated fatty acids (LCPUFA) was found to inhibit lymphocyte adhesion to vascular endothelial cells. (Khalfoun, B., et al. Transplantation (1996) 62:1649-1657). Subsequent investigations determined that administration of LCPUFA reduces endothelial cell expression of adhesion molecules and cytokines in response to stimulation, suggesting that LCPUFA have anti-atherogenic and anti-inflammatory properties. (De Caterina, R., et al. Am. J. Clin Nutr. (2000) 71(suppl)213S-223S). Mounting evidence indicates that regular consumption of LCPUFA, in particular, n-3 fatty acids, protects the cardiovascular system, reduces atherosclerotic plaque formation, and reduces the risk of mortality from cardiovascular disease, particularly following a myocardial infarction. (Calder, P.C. Clin. Sci. (Lond). 2004 107:1-11). In contrast, there has been little investigation of the effect of dietary LCPUFA on the vasculature and tissue of the brain, particularly with respect to an ischemic episode in the brain.

It has long been known that oxidative stress and damage is involved in the aging process. Oxidative stress has also been established as playing a role in the pathogenesis of a number of neurodegenerative diseases, including Alzheimer's disease and certain forms of dementia (e.g., Smith, M.A., Dementia & Geriatric Cognitive Disorders (1999), Vol. 10, Supp.1). Accordingly, supplementation with antioxidants has been employed to retard the aging process and protect against the degenerative effects of oxidative stress. Likewise, the B vitamins folic acid, vitamin B-6, and vitamin B-12 are important for the well-being and normal functioning of the brain. The status of these vitamins is frequently inadequate in the elderly, and these inadequacies can result in hyperhomocysteinemia, a recently identified risk factor for atherosclerosis (Temple ME, et al, Ann Pharmacother 2000; 34: 57-65.; Hankey GJ, et al., Lancet 1999; 354:407-413) and Alzheimer's disease (Clarke R, et al., Arch Neurol 1998 55:1449-1455). These inadequacies can result in ischemia of the brain or other tissues *via* occlusive vascular disease, stroke, or thrombosis.

DE 10214005 discloses a composition which comprises 5-15 g pectin and/or guar and 1-3 g EPA/DHA in a preferred ratio of 2 to 3, whereby the omega-3 fatty acids are stabilized against oxidation with vitamin C and/or E and before being mixed with pectin/guar they are preferably flavoured with caramel or vanilla. The composition disclosed may also include 5g arginine. The document discloses that the omega-3 fatty acids have a positive affect on blood pressure, heart arrythmia, cardiac infarct, risk of stroke, arteriosclerosis, and on inflammatory diseases such as rheumatism, psoriasis, arturistis, ulcerative celitis and Crohn's disease.

US 2002/0182196 relates to a composition and method for normalising impared or deteriorating neurological function. The document discloses that

impaired or deteriorating neurological functions are manifested by a variety of conditions spanning a spectrum of states of nervous system dysfunction. Non-limiting examples of such conditions include memory deterioration, certain behavioral problems, attention deficit disorder (ADD), attention deficit hyperactivity disorder (ADHD), other inattention and/or hyperactivity syndromes, dementia or cognitive decline of multiple etiologies, various genetic disorders (e.g., Downs Syndrome, Fragile X Syndrome, etc.), central nervous system (CNS) trauma, intoxications (acute and chronic) or poisoning, auto-immune mechanisms, anoxic-ischemic conditions, neurodegenerative disorders, metabolic afflictions of the nervous system, vascular insults, hypertensive encephalopathy, rheological disorders, demyelination, cerebral edema, inflammatory neuronal conditions, learning disabilities, impulsive behavior, specific emotional or mood problems, difficulty functioning under pressure, various latrogenic conditions, infections, eleptogenic foci and congenital brain malformations.

The document discloses that the composition contains effective amounts of
(A) at least one agent which promotes synthesis of adenosine triphosphate (ATP) and/or creatine phosphate in the body;
(B) at least one antioxidant for scavenging free radicals in at least one pathway in the body;
(C) at least one agent for normalizing or maintaining membrane function and structure in the body;
(D) at least one agent for normalizing or maintaining normal neurotransmitter function in the body;
(E) at least one agent for down-regulating cortisol action; and
(F) at least one agent for suppressing activation of apoptotic pathways in the body; with possible inclusion of one or more of the following:
(G) at least one agent for suppressing inflammation in the body,
(H) at least one agent for normalizing or maintaining vascular wall function and structure in the body;
(I) at least one agent for normalizing or maintaining function of nerve growth factors and/or neurotropic factors in the body:
(J) at least one agent for suppressing toxic metal ionic effects;
(K) at least one agent for normalizing or maintaining methyl metabolism in the body;
(L) at least one agent for normalizing or maintaining metabolism of insulin and glucose in the body; and
(M) at least one agent for up-regulating activity of heat shock proteins in the body.
(B) and (H) can include arginine and
(C) can include gamma linolenic acid and high polyunsaturated LCPUFAS such as DHA.

### SUMMARY OF THE INVENTION

The invention provides uses and compositions for use as defined in the appended claims. Described herein are compositions comprising one or more long chain polyunsaturated fatty acids (LCPUFA) and one or more nitric oxide releasing compounds (NORC) in an amount effective for the enhancement of vascular integrity, for example in the brain, and/or reduction of apoptosis in an animal. The enhanced vascular integrity, for example of the brain, and/or cellular survival serves to reduce ischemic injury in tissues, for example the brain, in the event of an ischemic episode.

In certain embodiments, the composition is a pet food composition, dietary supplement, or a food product formulated for human consumption. In various embodiments, the LCPUFA include at least one of arachidonic acid, eicosapentaenoic acid, docosapentaenoic acid, or docosahexaenoic acid, and the NORC include at least one of L-arginine or derivatives thereof. The compositions may comprise additional ingredients. For example, one or more antioxidants, B-vitamins, cellular growth factors or anti-inflammatory agents may be included.

In certain embodiments, the compositions are formulated for companion animals, such as a dog or a cat. In other embodiments, the compositions are formulated for human consumption.

Described herein are methods for enhancing vascular integrity and or reducing ischemia-induced brain injury in an animal, comprising administering to the animal on a regular basis a composition comprising one or more LCPUFA and one or more NORC, optionally supplemented with additional ingredients as described above, in an amount effective to enhance the vascular integrity in tissue(s) of the animal and/ or reduce ischemia-induced brain injury in the event of an ischemic episode in the brain of the animal. In certain embodiments, the method is applied to a companion animal, such as a dog or a cat. In other embodiments, the method is applied to humans.

In certain embodiments, the regular administration of the composition to the animal results in a reduction in brain damage in the event of an ischemic episode in the brain of the animal. In another embodiment, the regular administration of the composition to the animal results in a reduction in cellular apoptosis at the location of an ischemia occurring in the animal, which may be in any tissue, including brain, heart and kidney.

Other features and advantages of the invention will become apparent by reference to the drawings, detailed description and examples that follow.

### BRIEF DESCRIPTION OF THE DRAWINGS

**Figure 1****.** Bar graph showing effect of control and test diets on ischemic lesion volume in all rats subjected to transient cerebral ischemia. Compositions of control and test diets are described in Example 1 (*p < 0.01 vs control).
**Figure 2****.** Bar graph showing effect of control and test diets on ischemic lesion volume in rats exhibiting lesions after transient cerebral ischemia. Compositions of control and test diets are described in Example 1 (*p < 0.01 vs control).
**Figure 3****.** Bar graph showing effect of control and test diets on cerebral ischemia-induced apoptosis in rats. Compositions of control and test diets are described in Example 1 (*p < 0.001 vs control).
**Figure 4****.** Graph showing correlation between ischemic lesion volume (X axis) and percentage of apoptotic cells as defined by TUNEL staining (Y axis) in rats subjected to transient cerebral ischemia. Compositions of control and test diets are described in Example 1.

### DETAILED DESCRIPTION OF ILLUSTRATIVE EMBODIMENTS

### Definitions

Various terms relating to the methods and other aspects of the present invention are used throughout the specification and claims. Such terms are to be given their ordinary meaning in the art unless otherwise indicated. Other specifically defined terms are to be construed in a manner consistent with the definition provided herein.

The following abbreviations may be used in the specification and examples: AA, arachidonic acid; ALA, alpha-linolenic acid; ANOVA, analysis of variance; DHA, docosahexaenoic acid; DM, dry matter; DPA, docosapentaenoic acid; EPA, eicosapentaenoic acid; LA, linoleic acid; LCPUFA, long chain polyunsaturated fatty acid; NORC, nitric oxide releasing compounds; L-Arg, L-arginine.

Within the context of this specification the term "about" is interpreted to mean optionally plus or minus 20%; more preferably optionally plus or minus 10%; even more preferably optionally plus or minus 5%; most preferably optionally plus or minus 2%.

"Effective amount" refers to an amount of a compound, material, or composition, as described herein that is effective to achieve a particular biological result. Such results include, but are not limited to, enhancing vascular integrity or reducing ischemic brain injury. Such effective activity may be achieved, for example, by administering the compositions of the present invention to the animal.

The term "vascular integrity" refers to the overall health of the blood vessels in an animal, including without limitation, healthy vascular tone and structure, healthy vascular growth, healthy constriction and dilation, diminished permeability, diminished proliferation, diminished inflammation, diminished cell and/or platelet adhesion, maintenance of normal blood pressure and blood flow, and the like. "Enhanced vascular integrity" refers to any improvement in the overall health of the blood vessels in an animal, as measured by any means suitable in the art.

As used herein, "long chain polyunsaturated fatty acids" or "LCPUFA" refers to any monocarboxylic acid having at least 20 carbon atoms and at least two double bonds. Non-limiting examples of LCPUFA include (n-6) fatty acids such as arachidonic acid, and (n-3) fatty acids such as eicosapentaenoic acid, docosapentaenoic acid and docosahexaenoic acid.

As used herein, "nitric oxide releasing compounds" or "NORC" refers to any compound that causes the release of free nitric oxide in an animal. Preferred examples of such compounds include L-arginine and analogs or derivatives of L-arginine such as arginine alpha-ketoglutarate, GEA 3175, sodium nitroprusside, glyceryl trinitrate, *S*-nitroso-*N*-acetyl-penicillamine, nitroglycerin, S-NO-glutathione, NO-conjugated non-steroidal anti-inflammatory drugs such as NO-naproxen, NO-aspirin, NO-ibuprofen, NO-Diclofenac NO-Flurbiprofen and NO-Ketoprofen, NO-releasing compound-7, NO-releasing compound-5, NO-releasing compound-12, NO-releasing compound-18, diazeniumdiolates and derivatives thereof, diethylamine NONOate, or any organic or inorganic compound, biomolecule, or analog, homolog, conjugate, or derivative thereof that causes the release of free nitric oxide in an animal.

"Ischemia" refers to any decrease or stoppage in the blood supply to any bodily organ, tissue, or part caused by any constriction or obstruction of the vasculature. "Ischemic episode" refers to any transient or permanent period of ischemia.

"Vasculature" refers to any network of blood vessels in the body of an animal, the blood vessels including without limitation arteries, veins, and capillaries.

"Vascular endothelial cells" or "vascular endothelium" refers to cells that comprise the layer of thin, flat cells that line the interior surface of blood vessels, forming an interface between circulating blood in the lumen and the rest of the vessel wall.

The present invention relates to any animal, preferably a mammal, more preferably companion animals, and most preferably humans. A "companion animal" is any domesticated animal, and includes, without limitation, cats, dogs, rabbits, guinea pigs, ferrets, hamsters, mice, gerbils, horses, cows, goats, sheep, donkeys, pigs, and the like.

As used herein, the term "pet food" or "pet food composition" means a composition that is intended for ingestion by an animal, and preferably by companion animals. A "complete and nutritionally balanced pet food," is one that contains all known required nutrients in appropriate amounts and proportions based on recommendations of recognized authorities in the field of companion animal nutrition, and is therefore capable of serving as a sole source of dietary intake to maintain life or promote production, without the addition of supplemental nutritional sources. Nutritionally balanced pet food compositions are widely known and widely used in the art.

As used herein, a "dietary supplement" is a product that is intended to be ingested in addition to the normal diet of an animal.

As used herein, a "food product formulated for human consumption" is any composition intended for ingestion by a human being.

### Description

The inventors have observed that supplemental arginine in dogs increased expression of multiple growth factors and anti-inflammatory proteins in a dose-dependent manner. Many such growth factors, including NGF, nIGF, and BDNF exert potent neuronal protective effects. (Mattson, MP et al. Neurobiol. Aging. (2002) 23:695-705, and Kruttgen, A. et al. Proc. Natl. Acad Sci. USA (1998) 95:9614-9619). In further investigations of dogs, using natural sources of arginine, including herring meal (containing fish oil rich in LCPUFA), the inventors found an equal or better response than that observed using pure L-arginine. As described in detail in the examples herein, functional studies of transient cerebral ischemia in an ovariectomized rat model showed that a diet containing a combination of L-arginine, fish oil, antioxidants, and B-vitamins markedly reduced cerebral lesions and apoptosis, more so than diets containing arginine or fish oil alone. Accordingly, various aspects of the present invention utilize these discoveries by providing dietary compositions and methods to improve vascular integrity of an animal and to reduce ischemic injury in the event of ischemia in the animal, for example a brain injury in the event of a brain ischemia in the animal.

The dietary compositions are expected to be effective in a variety of ischemic situations, including but are not limited to, brain aging (strokes and mini strokes) and related disorders, ischemic cardiac events, induced ischemia and reperfusion surgery (pre- and post-operative administration), diabetes-induced peripheral ischemia, and as a post-menopause alternative to estrogen treatment to reduce the risk of stroke and cardiovascular disease in postmenopausal women.

In various embodiments, the compositions and methods of the present invention apply a multi-component approach to the improvement of functional vascular integrity, reduction in apoptosis and enhancement of cellular survival after an ischemic occurrence. These components include (1) enhancing vascular integrity and function, (2) enhancing cellular repair, (3) improving anti-inflammatory effects (4) optimizing cellular metabolism, and (5) reducing oxidative stress.

### Compositions

Described herein are compositions comprising one or more long chain polyunsaturated fatty acids (LCPUFA) and one or more nitric oxide releasing compounds (NORC) in an amount effective for the enhancement of vascular integrity in animals. The LCPUFA and NORC can be present in the composition as an ingredient or additive. In preferred embodiments of the composition, the LCPUFA comprise at least one of the (n-3) fatty acids such as ALA, EPA, DPA and DHA, and the NORC comprise at least one of L-Arg and derivatives thereof. The compositions enrich the blood plasma with LCPUFA and NORC in animals to which the composition is administered.

The compositions of the invention may include a number of additional components for the purpose of providing the multi-component approach to promoting vascular integrity and enhancing cellular survival in ischemia, as outlined above. These include antioxidants, B-vitamins, growth factors and anti-inflammatory agents.

In one preferred embodiment, the compositions of the invention are pet food compositions. These will advantageously include foods intended to supply necessary dietary requirements, as well as treats (*e.g*., biscuits) or other dietary supplements. Optionally, the pet food compositions can be a dry composition (for example, kibble), semi-moist composition, wet composition, or any mixture thereof.

In another preferred embodiment, the compositions of the invention are food products formulated for human consumption. These will advantageously include foods and nutrients intended to supply necessary dietary requirements of a human being as well as other human dietary supplements. In a detailed embodiment, the food products formulated for human consumption are complete and nutritionally balanced.

In another preferred embodiment, the composition is a dietary supplement, such as a gravy, drinking water, beverage, liquid concentrate, yogurt, powder, granule, paste, suspension, chew, morsel, treat, snack, pellet, pill, capsule, tablet, or any other delivery form. The dietary supplements can be specially formulated for consumption by a particular animal, such as companion animal, or a human. In one detailed embodiment, the dietary supplement can comprise a high concentration of LCPUFA and NORC such that the supplement can be administered to the animal in small amounts, or in the alternative, can be diluted before administration to an animal. The dietary supplement may require admixing with water prior to administration to the animal.

The composition may be frozen. The LCPUFA and NORC may be pre-blended with the other components of the composition to provide the beneficial amounts needed, may be coated onto a pet food composition, dietary supplement, or food product formulated for human consumption, or may be added to the composition prior to offering it to the animal, for example, using a powder or a mix.

The compositions of the invention comprise LCPUFA and NORC in an amount effective to enhance vascular integrity in an animal to which the composition has been administered. For pet foods and food products formulated for human consumption, the amount of LCPUFA as a percentage of the composition is in the range of about 0.1% to about 13% of the composition on a dry matter basis, although a greater percentage can be supplied. In various embodiments, the amount is about 0.1%, 0.2%, 0.3%, 0.4%, 0.5%, 0.6%, 0.7%, 0.8%, 0.9%, 1.0%, 1.1%, 1.2%, 1.3%, 1.4%, 1.5%, 1.6%, 1.7%, 1.8%, 1.9%, 2.0%, 2.1%, 2.2%, 2.3%, 2.4%, 2.5%, 2.6%, 2.7%, 2.8%, 2.9%, 3.0%, 3.1 %, 3.2%, 3.3%, 3.4%, 3.5%, 3.6%, 3.7%, 3.8%, 3.9%, 4.0%, 4.1 %, 4.2%, 4.3%, 4.4%, 4.5%, 4.6%, 4.7%, 4.8%, 4.9%, 5.0%, or more, e.g., 6%, 7%, 8%, 9%, 10%, 11%, 12%, 13% or more, of the composition on a dry matter basis, and the amount of NORC as a percentage of the composition is in the range of about 0.1 % to about 12% of the composition on a dry matter basis, although a greater percentage can be supplied. In various embodiments, the amount is about 0.1%, 0.2%, 0.3%, 0.4%, 0.5%, 0.6%, 0.7%, 0.8%, 0.9%, 1.0%, 1.1 %, 1.2%, 1.3%, 1.4%, 1.5%, 1.6%, 1.7%, 1.8%, 1.9%, 2.0%, 2.1%, 2.2%, 2.3%, 2.4%, 2.5%, 2.6%, 2.7%, 2.8%, 2.9%, 3.0%, 3.1 %, 3.2%, 3.3%, 3.4%, 3.5%, 3.6%, 3.7%, 3.8%, 3.9%, 4.0%, 4.1 %, 4.2%, 4.3%, 4.4%, 4.5%, 4.6%, 4.7%, 4.8%, 4.9%, 5.0%, or more, e.g., 6%, 7%, 8%, 9%, 10%, 11 %, 12% or more, of the composition on a dry matter basis. In a specific embodiment, 2-2.5% LCPUFA and 2-2.5% pure L-arginine are utilized. Dietary supplements may be formulated to contain several fold higher concentrations of LCPUFA and NORC, to be amenable for administration to an animal in the form of a tablet, capsule, liquid concentrated, or other similar dosage form, or to be diluted before administrations, such as by dilution in water, spraying or sprinkling onto a pet food, and other similar modes of administration.

In an alternative embodiment, the amount of LCPUFA and NORC in the composition is a function of an amount required to establish a specified concentration of LCPUFA and NORC in the blood serum of the animal. The specified concentration of LCPUFA in the blood serum may be calculated by determining the blood serum levels of animals fed the recommended amounts of LCPUFA and NORC specified above, as would be appreciated by one of skill in the art.

The sources of each of the LCPUFA can be any suitable source, synthetic or natural. Preferred sources of LCPUFA include, without limitation, primrose, dark green vegetables such as spinach, algae and blue-green algae such as spirulina, plant seeds and oils such as flaxseed, canola, soybean, walnut, pumpkin, safflower, sesame, wheat germ, sunflower, corn, and hemp, and fish, especially cold-water fish such as salmon, tuna, mackerel, herring, sea bass, striped bass, shark, halibut, catfish, sardines, shrimp, and clams, and their extracted oils, or the LCPUFA may be synthesized *de novo* according to any means suitable in the art.

The sources of each NORC can be any suitable source, synthetic or natural. Preferred sources of arginine include, without limitation, animal and plant proteins. Non-limiting examples of plants particularly rich in arginine content include legumes such as soy, lupins, and carob, grains such as wheat and rice, and fruits such as grapes. Seeds and nuts such as cacao and peanuts are also rich in arginine content. Non-limiting examples of animal proteins particularly rich in arginine content include poultry and fish products. The NORC can be synthesized *de novo,* according to any means suitable in the art, or provided by a commercial source.

The compositions of the invention can optionally comprise supplementary substances such as minerals, vitamins, salts, condiments, colorants, and preservatives. Non-limiting examples of supplementary minerals include calcium, phosphorous, potassium, sodium, iron, chloride, boron, copper, zinc, magnesium, manganese, iodine, selenium and the like. Non-limiting examples of supplementary vitamins include vitamin A, various B vitamins, vitamin C, vitamin D, vitamin E, and vitamin K. Additional dietary supplements may also be included, for example, niacin, pantothenic acid, inulin, folic acid, biotin, amino acids, and the like.

The compositions of the invention can optionally comprise one or more supplementary substances that promote or sustain vascular health, or further enhance vascular integrity or cellular survival. Such substances include, without limitation, one or more of vinpocetine, coenzyme Q₁₀, phosphatidylserine, acetyl-L-carnitine, alpha-lipoic acids, extracts such as Bilberry *(Vaccinium myrtilis),* or antioxidants, including tocopherols, tocotrienols, carotenoids such as alpha- and beta-carotene, lycopene, lutein, astaxanthin, zeaxanthine and flavonoids such as flavanols, flavanones, flavones, flavan-3-ols (catechins), anthocyanins, and isoflavones (isoflavonoids), and the like, as well as anti-inflammatory agents and growth factors (particularly applicable to promoting cellular survival), as would be understood by one of skill in the art.

In various embodiments, pet food or dietary supplement compositions of the invention can comprise, on a dry matter basis, from about 15% to about 50% crude protein, by weight of the composition. The crude protein material may comprise vegetable proteins such as soybean, cottonseed, and peanut, or animal proteins such as casein, albumin, and meat protein. Non-limiting examples of meat protein useful herein include pork, lamb, equine, poultry, fish, and mixtures thereof.

The compositions may further comprise, on a dry matter basis, from about 5% to about 40% fat, by weight of the composition. The compositions may further comprise a source of carbohydrate. The compositions may comprise, on a dry matter basis, from about 15% to about 60% carbohydrate, by weight of the composition. Non-limiting examples of such carbohydrates include grains or cereals such as rice, corn, sorghum, alfalfa, barley, soybeans, canola, oats, wheat, and mixtures thereof. The compositions may also optionally comprise other materials such as dried whey and other dairy by-products.

The compositions may also comprise at least one fiber source. A variety of soluble or insoluble fibers may be utilized, as will be known to those of ordinary skill in the art. The fiber source can be beet pulp (from sugar beet), gum arabic, gum talha, psyllium, rice bran, carob bean gum, citrus pulp, pectin, fructooligosaccharide additional to the short chain oligofructose, mannanoligofructose, soy fiber, fiber from lupins, arabinogalactan, galactooligosaccharide, arabinoxylan, or mixtures thereof. Alternatively, the fiber source can be a fermentable fiber. Fermentable fiber has previously been described to provide a benefit to the immune system of a companion animal. Fermentable fiber or other compositions known to those of skill in the art which provide a prebiotic composition to enhance the growth of probiotic microorganisms within the intestine may also be incorporated into the composition to aid in the enhancement of the benefit provided by the present invention to the immune system of an animal. Additionally, probiotic microorganisms, such as *Lactobacillus* or *Bifidobacterium* species, for example, may be added to the composition.

In a detailed embodiment, the composition is a complete and nutritionally balanced pet food. In this context, the pet food may be a wet food, a dry food, or a food of intermediate moisture content, as would be recognized by those skilled in the art of pet food formulation and manufacturing. "Wet food" describes pet food that is typically sold in cans or foil bags, and has a moisture content typically in the range of about 70% to about 90%. "Dry food" describes pet food which is of a similar composition to wet food, but contains a limited moisture content, typically in the range of about 5% to about 15%, and therefore is presented, for example, as small biscuit-like kibbles. The compositions and dietary supplements may be specially formulated for adult animals, or for older or young animals, for example, a "puppy chow," "kitten chow," or "senior" formulation. In general, specialized formulations will comprise energy and nutritional requirements appropriate for animals at different stages of development or age.

Certain aspects of the invention are preferably used in combination with a complete and balanced food (for example, as described in National Research Council, 1985, Nutritional Requirements for Dogs, National Academy Press, Washington D.C., or Association of American Feed Control Officials, Official Publication 1996). That is, compositions comprising LCPUFA, or DHA according to certain aspects of this invention are preferably used with a high-quality commercial food. As used herein, "high-quality commercial food" refers to a diet manufactured to produce the digestibility of the key nutrients of 80% or more, as set forth in, for example, the recommendations of the National Research Council above for dogs. Similar high nutrient standards would be used for other animals.

The skilled artisan will understand how to determine the appropriate amount of LCPUFA and NORC to be added to a given composition. Such factors that may be taken into account include the type of composition (*e.g*., pet food composition, dietary supplement, or food product formulated for human consumption), the average consumption of specific types of compositions by different animals, and the manufacturing conditions under which the composition is prepared. Preferably, the concentrations of LCPUFA and NORC to be added to the composition are calculated on the basis of the energy and nutrient requirements of the animal. According to certain aspects of the invention, the LCPUFA and NORC can be added at any time during the manufacture and/or processing of the composition. This includes, without limitation, as part of the formulation of the pet food composition, dietary supplement, or food product formulated for human consumption, or as a coating applied to the pet food composition, dietary supplement, or food product formulated for human consumption.

The compositions can be made according to any method suitable in the art such as, for example, that described in Waltham Book of Dog and Cat Nutrition, Ed. ATB Edney, Chapter by A. Rainbird, entitled "A Balanced Diet" in pages 57 to 74, Pergamon Press Oxford.

### Methods

Described herein are methods for enhancing the vascular integrity and/or promoting cellular survival in an animal comprising administering to the animal a composition comprising one or more LCPUFA and one or more NORC in an amount effective to enhance vascular integrity and / or promote cellular survival in the animal. Yet another aspect of the invention features prophylactic methods for reducing ischemia-induced injury to the brain or other tissues of an animal comprising administering to the animal on a regular basis a composition comprising one or more LCPUFA and one or more NORC in an amount effective to reduce ischemia-induced injury, for example to the brain, in the event of an ischemic episode in the animal, for example in the brain of the animal.

In detailed embodiments of either of the two above-mentioned aspects of the invention, the composition is a pet food composition, dietary supplement, or food product formulated for human consumption as exemplified herein. In a further detailed embodiment, the LCPUFA is one or more of an (n-3) LCPUFA, including but not limited to EPA, DPA and DHA, and the NORC is one or more of L-Arg and derivatives thereof. Animals can include any domesticated or companion animals as described above, or can include humans. In certain embodiments, the animal is a companion animal such as a dog or cat. In another embodiment, the animal is a human.

The compositions can be administered to the animal by any of a variety of alternative routes of administration. Such routes include, without limitation, oral, intranasal, intravenous, intramuscular, intragastric, transpyloric, subcutaneous, rectal, and the like. Preferably, the compositions are administered orally. As used herein, the term "oral administration" or "orally administering" means that the animal ingests or a human is directed to feed, or does feed, the animal one or more of the inventive compositions described herein.

Wherein the human is directed to feed the composition, such direction may be that which instructs and/or informs the human that use of the composition may and/or will provide the referenced benefit, for example, the enhancement of vascular integrity in the animal or cellular survival in the animal, reduction of ischemia-induced injury, for example to the brain, heart or other tissues, protection from ischemia-related damage in the event of an ischemic episode, for example in reperfusion surgery, diabetes-induced peripheral ischemia and others, as elaborated above. Such direction may be oral direction (*e.g*., through oral instruction from, for example, a physician, veterinarian, or other health professional, or radio or television media (i.e., advertisement), or written direction (*e.g*., through written direction from, for example, a physician, veterinarian, or other health professional (*e.g*., prescriptions), sales professional or organization (*e.g*., through, for example, marketing brochures, pamphlets, or other instructive paraphernalia), written media (*e.g*., internet, electronic mail, or other computer-related media), and/or packaging associated with the composition (*e.g*., a label present on a container holding the composition).

Administration can be on an as-needed or as-desired basis, for example, once-monthly, once-weekly, daily, or more than once daily. Similarly, administration can be every other day, week, or month, every third day, week, or month, every fourth day, week, or month, and the like. Administration can be multiple times per day. When utilized as a supplement to ordinary dietetic requirements, the composition may be administered directly to the animal or otherwise contacted with or admixed with daily feed or food. When utilized as a daily feed or food, administration will be well known to those of ordinary skill.

Administration can also be carried out on a regular basis, for example, as part of a diet regimen in the animal. A diet regimen may comprise causing the regular ingestion by the animal of a composition comprising one or more LCPUFA and one or more NORC in an amount effective to enhance vascular integrity or to reduce ischemia-induced injury to the brain in the event of an ischemic episode in the animal. Regular ingestion can be once a day, or two, three, four, or more times per day, on a daily or weekly basis. Similarly, regular administration can be every other day or week, every third day or week, every fourth day or week, every fifth day or week, or every sixth day or week, and in such a dietary regimen, administration can be multiple times per day. The goal of regular administration is to provide the animal with the preferred daily dose of LCPUFA and NORC, as exemplified herein.

Administration of the compositions comprising one or more LCPUFA and one or more NORC, including administration as part of a diet regimen, can span a period of time ranging from gestation through the entire life of the animal.

The following examples are provided to describe the invention in greater detail. They are intended to illustrate, the invention.

### Example 1

### Effect of 17 β-estradiol or dietary supplementation with arginine, fish oil or combination on brain damage from transient cerebral ischemia in ovariectomized rat model

Chronic treatment with 17β-estradiol (E2) was compared with three test diets containing arginine, LCPUFA in the form of fish oil, or a combination thereof, respectively, for their effects on brain damage caused by transient cerebral ischemia in an ovariectomized rat model.

### Methods:

**Animals**. Female Charles Rivers Sprague-Dawley rats (250g, Wilmington, MA) were acclimatized to animal facilities three days prior to surgery with a 12 hour light-dark cycle. Bilateral ovariectomy was performed 2 weeks before diet feeding began. Four weeks after the start of diet feeding, transient middle cerebral artery (tMCA) occlusion under anesthesia was performed following intraperitoneal (i.p.) injection of ketamine (60mg/kg) and xylazine (10 mg/kg).

**Diet and Hormone Administration**. Fourteen to 15 rats per group were randomly assigned to one of five treatment groups. These groups were as follows:
Group 1 Control diet (White Diet)
Group 2 Control diet + SILASTIC® estradiol implant for 1 week (White + E2)
Group 3 Diet I (Pink Diet)
Group 4 Diet II (Purple Diet)
Group 5 Diet III (Gray Diet)

The control diet was a standard rat diet containing 140 g/kg casein, 100 g/kg sucrose, 50 g/kg fiber, 155 g/kg dextrin, 466 g/kg corn starch, 35 g/kg standard salt mix, 40 g/kg soybean oil, 10 g/kg standard vitamin mix, 1.8 g/kg L-cystine and 2.5 g/kg choline chloride. All three test diets (Diets I, II and III) were supplemented with (1) additional B vitamins, including 45 mg/kg nicotinic acid, 35 mg/kg pantothenate, 24 mg/kg pyridoxine, 15 mg/kg thiamin, 9 mg/kg riboflavin 3 mg/kg folic acid, 0.8 mg/kg biotin and 0.225 mg/kg vitamin B12, and (2) an antioxidant cocktail comprising 500 mg/kg vitamin E, 150 mg/kg vitamin C, 100 mg/kg astaxanthin and 0.4 mg/kg selenium. Diet I further included 2% arginine (20 g/kg). Diet II further included 2% arginine and 2% menhaden fish oil (20 g/kg). Diet III further included 2% menhaden fish oil.

All diets were fed *ad libitum* to the rats for four weeks. Food changes occurred once per week. Also once per week, body weights and food intake were determined. One week before the induction of a tMCA occlusion, Group 2 rats were administered E2 at a dose of 4 mg/ml in a SILASTIC® pellet that was implanted subcutaneously. This procedure has been used to protect the brain from a variety of insults and produces physiologically relevant level of serum E2 (Simpkins et al., 1997; Jung et al., 2003). This was a positive control, since it has been shown previously that this dose and time of E2 treatment resulted in protection from the effects of tMCA occlusion (Simpkins, et al., 1997; Yang et al., 2004a, Yang, et al., 2004b).

**Middle cerebral artery occlusion and samples preparation. Animals were** anesthetized by an intraperitoneal injection of ketamine (60 mg/kg) and xylazine (10mg/kg). tMCA occlusion was performed as previously described (Simpkins et al., 1997) with slight modification. Briefly, the left common carotid artery, external carotid artery and internal carotid artery were exposed through a midline cervical incision. A 3.0 mono-filament suture was introduced into the internal carotid artery lumen and gently advanced until resistance was felt. The surgical procedure was performed within 20 minutes, with minimal bleeding. The suture was kept in place for 60 minutes and then withdrawn to allow reperfusion. Rectal temperature was monitored and maintained between 36.5 and 37°C with a heating pad throughout the procedure. At 24 hours after the onset of reperfusion, the animals were sacrificed and the brains were removed. The brains were then dissected coronally into 2mm sections using a metallic brain matrix (ASI Instruments Inc.; Warren, MI) and stained by incubation in a 2% solution of 2,3,5-triphenyltetrazolium chloride (TTC) in physiological saline at 37°C, and then fixed in 10% formalin.

DNA fragmentation analysis with TdT-mediated dUTP Nick-End Labeling (TUNEL) MCA occlusion is a widely used focal ischemic stroke model (Bederson et al., 1986). This in vivo model for neuronal death can rapidly induce a synchronized apoptotic process in a large number of neurons and other cells (Li et al., 1997). The effects of transient ischemia on apoptosis were therefore examined by analyzing DNA fragmentation with the TUNEL assay. TUNEL staining was performed according to the modified manufacturer's instructions (Gavrieli et al., 1992). Formalin-fixed, paraffin-embedded tissue sections were deparaffinizated with xylene, rehydrated through graded ethanol treatment, and given a final wash in PBS. The sections were post-fixed in 4% paraformaldehyde for 20 minutes. Sections were then washed and treated for 15 minutes with 100/µg/ml proteinase K in PBS, equilibrated with equilibration buffer for 10 min, and then incubated with buffer containing TdT enzyme and FITC-labeled dUTP (Promega, Madison, WI) at 37°C in a humidified chamber. The reaction was terminated by incubation in 2xSSC buffer for 15 min at room temperature. The sections were then mounted with anti-fade reagents containing DAPI (Molecular Probes, Eugene, OR). Positive control sections were immersed in DNase I solution for 10 min at room temperature before equilibration in TdT buffer. The sections were observed under a fluorescent microscope with appropriate excitation/emission filter pairs.

Some animals were eliminated from the study during the protocol. The number of animals quantified for lesion volume ranged from 12 to 15 per group.

**Statistical methods.** Results were analyzed with one-way analysis of variance (ANOVA) using Prism software(Graphpad Inc; San Diego, CA). The significance of differences among groups was determined by one-way ANOVA followed by Tukey's multiple comparison tests. All values were expressed as mean +/- SEM.

### Results:

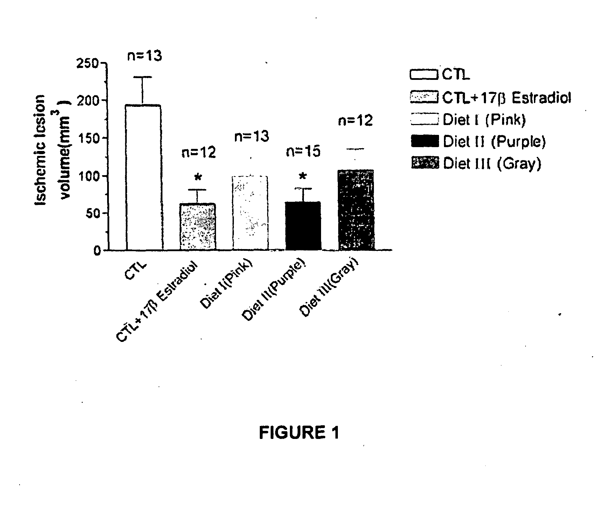

**Stroke Volume.** All four experimental conditions (estradiol and Diets I, II and III) reduced infarct size (**Figure 1**)**.** Estradiol treatment (Group 2) reduced infarct volume by 68%, a value typical of an estrogen protection from stroke damage (Simpkins, et al., 1997; Fan et al., 2003; Yang et al., 2004a; Yang et al., 2004b). Similarly, Diet II reduced infarct volume significantly, by 67%. The two other diets tested also reduced mean infarct volumes, but the data were too variable for the values to be statistically significant.

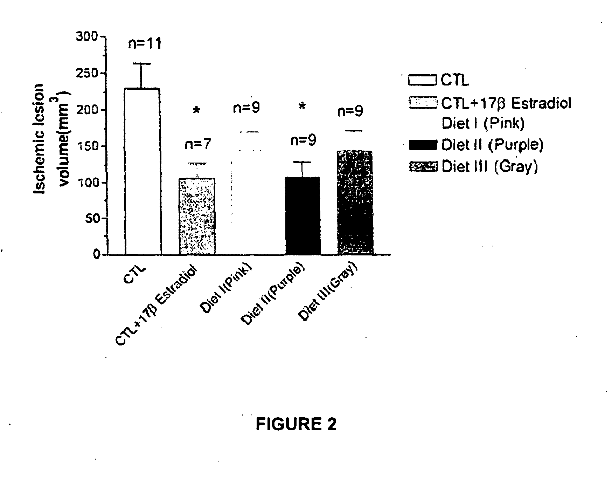

Inasmuch as each group had rats with no observable lesions (value of 0 in our calculations of lesion volume), the data were assessed after these values were omitted, to determine the extent to which the 0 values contributed to the group differences. As shown in **Figure 2****,** the same two groups were significantly lower than the control diet group with this modification in the data. Thus, the differences among groups were driven by the animal in which lesions were observed as well as by the number of animals without observable lesions. The protective effects of both estrogen treatment and diets were exerted primarily on the cortex, an area of the brain called the penumbra, as it is believed to be "rescueable" in experimental stroke. This is in contrast to the basal ganglia, called the core of the infarct, which is not readily saved by any treatment tested to date in experimental stroke.

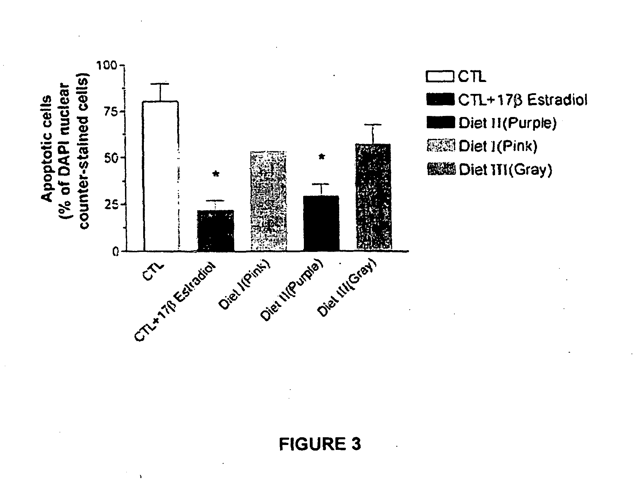

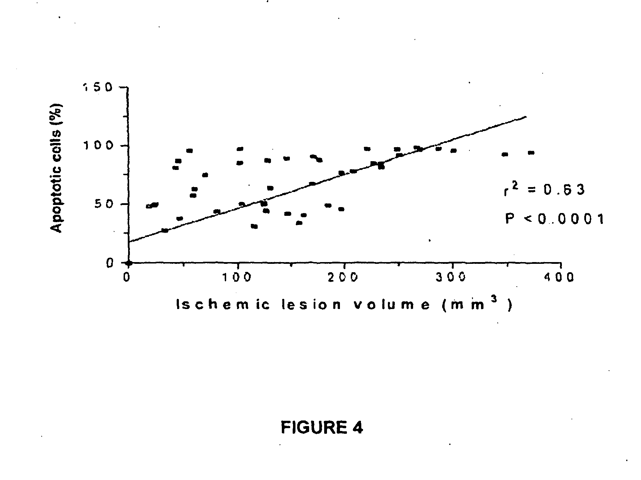

**Apoptosis**. Assessment of apoptosis using TUNEL staining was conducted to determine the consequences of estradiol treatment and the three test diets on the apoptotic response to experimental ischemia. Three brain regions for TUNEL staining were assessed in all animals that completed the study. TUNEL was assessed in the core of the cerebral cortex, the penumbra of the cerebral cortex and the core of the subcortex, based upon our assessment of damaged brain regions using TTC staining (Wen et al., 2004). The number of TUNEL positive cells was normalized to the total number of cells in the field using DAPI nuclear counter staining. Sections from all three brain regions were counted and their average cell counts for each animal were used to generate the group means. Cell counts were conducted in randomly selected microscopic fields (320 µm square sections) in slices from the maximum extent of the brain infarct, as determined by TTC staining. TUNEL staining was extensive in all three brain regions in the control animals. When a comparison of all five treatment groups was done, the TUNEL staining in each brain correlated with the results found using TTC staining to quantify lesion volume **(****Figure 3****).** That is, there was a substantial reduction in TUNEL staining in both the E2-treated animals as well as animals fed Diet II. Animals fed Diets I and III were intermediate between controls and the Diet II animals. This correlation between TTC staining and TUNEL staining is depicted in **Figure 4****.** The characterization of the effects of hormones and diets on TUNEL staining indicates that a substantial portion of the protection by E2 and Diet II from cell death is mediated by an inhibition of apoptosis.

### Conclusion:

All three test diets reduced mean infarct volume as well as TUNEL-positive cell counts and Diet II reduced these parameters to the level seen with a known neuroprotectant, estradiol.

## Claims

1. Use of one or more long chain polyunsaturated fatty acid (LCPUFA) and one or more nitric oxide releasing compound (NORC) in the manufacture of a medicament or medicaments for co-administration for enhancing vascular integrity are reducing cellular apoptosis at the location of an ischemia in an animal.

2. The use of claim 1, wherein the ischemia occurs in a tissue selected from brain, heart and kidney.

3. Use of one or more LCPUFA and one or more NORC in the manufacture of a medicament or medicaments for co-administration for reducing ischemia-induced brain injury in an animal.

4. Use according to any of claims 1 to 3, wherein the medicament is a pet food composition, dietary supplement, or a food product formulated for human consumption.

5. Use according to any of claims 1 to 3, wherein the LCPUFA include at least one of arachidonic acid, eicosapentaenoic acid, docosapentaenoic acid, or docosahexaenoic acid.

6. Use according to any of claims 1 to 3, wherein the NORC include at least one of L-arginine or derivatives thereof.

7. Use according to claim 6, wherein the LCPUFA are in an amount of at least about 0.1 % to about 13% by weight of the formulation and L-arginine in an amount of at least about 0.1 % to about 12% by weight of the formulation.

8. Use according to any of claims 1 to 3, wherein the animal is a companion animal.

9. Use according to claim 8, wherein the companion animal is a cat or dog.

10. Use according to any of claims 1 to 3, wherein the animal is a human.

11. A composition comprising one or more LCPUFA and one or more NORC for use in reducing ischemia-induced brain injury in an animal, wherein the composition is administered to the animal on a regular basis.

12. The composition of claim 11 wherein the composition is a pet food composition, dietary supplement, or a food product formulated for human consumption.

13. The composition of claim 11 wherein the LCPUFA include at least one of arachidonic acid, eicosapentaenoic acid, docosapentaenoic acid, or docosahexaenoic acid.

14. The composition of claim 11 wherein the NORC include at least one of L-arginine or derivatives thereof.

15. The composition of claim 14, wherein the LCPUFA are in an amount of at least about 0.1% to about 13% by weight of the formulation and L-arginine in an amount of at least about 0.1% to about 12% by weight of the formulation.

16. The composition of claim 11, wherein the animal is a companion animal.

17. The composition of claim 16, wherein the companion animal is a cat or dog.

18. The composition of claim 11, wherein the animal is a human.

19. A composition comprising one or more LCPUFA and one or more NORC for use in enhancing vascular integrity and reducing brain damage in the event of an ischemic episode in the brain.

20. A composition comprising one or more LCPUFA and one or more NORC for use in enhancing vascular integrity and reducing cellular apoptosis at the location of an ischemia in an animal.

21. The composition of claim 20, wherein the ischemia occurs in a tissue selected from brain, heart and kidney.

22. A composition comprising one or more LCPUFA and one or more NORC for use in reducing ischemia-induced brain injury in an animal.

## Patentansprüche

1. Gebrauch einer oder mehrerer langkettiger mehrfach ungesättigter Fettsäuren (LCPUFA) und einer oder mehrerer Stickoxid freisetzenden Verbindungen (NORC) in der Herstellung eines Medikaments oder Medikamente zur Mitverabreichung zur Verbesserung der Gefäßintegrität und Reduzierung zellulärer Apoptose am Ort einer Ischämie in einem Tier.

2. Der Gebrauch nach Anspruch 1, wobei die Ischämie in einem Gewebe ausgewählt aus Gehirn, Herz oder Niere auftritt.

3. Gebrauch einer oder mehrerer LCPUFA und einer oder mehrerer NORC in der Herstellung eines Medikaments oder Medikamente zur Mitverabreichung zur Reduzierung ischämie-induzierter Gehirnschädigung in einem Tier.

4. Gebrauch nach allen Ansprüchen 1 bis 3, wobei das Medikament eine Haustiernahrungszusammensetzung, diätetisches Ergänzungsmittel oder ein Nahrungsmittelprodukt ist, das zum menschlichen Verzehr hergestellt ist.

5. Gebrauch nach allen Anprüchen 1 bis 3, wobei die LCPUFA mindestens eine der Arachidonsäure, Eicosapentaensäure, Docosapentaensäure oder Docosahexaensäure beinhaltet.

6. Gebrauch nach allen Ansprüchen 1 bis 3, wobei die NORC mindestens ein L-Arginin oder Derivate davon beinhaltet.

7. Gebrauch nach Anspruch 6, wobei die LCPUFA in einer Menge von mindestens etwa 0,1 % bis etwa 13 % des Rezepturgewichts vorliegen und L-Arginin in einer Menge von mindestens etwa 0,1 % bis etwa 12 % des Rezepturgewichts.

8. Gebrauch nach allen Ansprüchen 1 bis 3, wobei das Tier ein Haus-/Nutztier ist.

9. Gebrauch nach Anspruch 8, wobei das Haus-/Nutztier eine Katze oder ein Hund ist.

10. Gebrauch nach allen Ansprüchen 1 bis 3, wobei das Tier ein Mensch ist.

11. Eine Zusammensetzung, umfassend eine oder mehrere LCPUFA und eine oder mehrere NORC für den Gebrauch zur Reduzierung ischämie-induzierter Gehirnschädigung in einem Tier, wobei die Zusammensetzung dem Tier regelmäßig verabreicht wird.

12. Die Zusammensetzung nach Anspruch 11, wobei die Zusammensetzung eine Haustiernahrungszusammensetzung, diätetisches Ergänzungsmittel oder ein Nahrungsmittelprodukt ist, das zum menschlichen Verzehr hergestellt ist.

13. Die Zusammensetzung nach Anspruch 11, wobei die LCPUFA mindestens eine der Arachidonsäure, Eicosapentaensäure, Docosapentaensäure oder Docosahexaensäure beinhaltet.

14. Die Zusammensetzung nach Anspruch 11, wobei die NORC mindestens ein L-Arginin oder Derivate davon beinhaltet.

15. Die Zusammensetzung nach Anspruch 14, wobei die LCPUFA in einer Menge von mindestens etwa 0,1 % bis etwa 13 % des Rezepturgewichts vorliegen und L-Arginin in einer Menge von mindestens etwa 0,1 % bis etwa 12 % des Rezepturgewichts.

16. Die Zusammensetzung nach Anspruch 11, wobei das Tier ein Haus-/Nutztier ist.

17. Die Zusammensetzung nach Anspruch 16, wobei das Haus-/Nutztier eine Katze oder ein Hund ist.

18. Die Zusammensetzung nach Anspruch 11, wobei das Tier ein Mensch ist.

19. Eine Zusammensetzung, umfassend eine oder mehrere LCPUFA und eine oder mehrere NORC für den Gebrauch zur Verbesserung der Gefäßintegrität und zur Reduzierung einer Gehirnschädigung während des Ereignisses eines ischämischen Vorfalls im Gehirn.

20. Eine Zusammensetzung, umfassend eine oder mehrere LCPUFA und eine oder mehrere NORC für den Gebrauch zur Verbesserung der Gefäßintegrität und zur Reduzierung zellulärer Apoptose am Ort einer Ischämie in einem Tier.

21. Die Zusammensetzung nach Anspruch 20, wobei die Ischämie in einem Gewebe ausgewählt aus Gehirn, Herz oder Niere auftritt.

22. Eine Zusammensetzung, umfassend eine oder mehrere LCPUFA und eine oder mehrere NORC für den Gebrauch zur Reduzierung einer ischämieinduzierten Gehirnschädigung in einem Tier.

## Revendications

1. Utilisation d'un ou plusieurs acides gras polyinsaturés à chaîne longue (LCPUFA) et un ou plusieurs composés libérant de l'oxyde nitrique (NORC) dans la fabrication d'un médicament ou de médicaments pour une coadministration destinés à améliorer l'intégrité vasculaire et à réduire l'apoptose cellulaire à l'emplacement d'une ischémie chez un animal.

2. Utilisation selon la revendication 1, où l'ischémie survient dans un tissu choisi parmi le cerveau, le coeur et le rein.

3. Utilisation d'un ou plusieurs LCPUFA et d'un ou plusieurs NORC dans la fabrication d'un médicament ou de médicaments pour une coadministration destinés à réduire une lésion cérébrale induite par une ischémie chez un animal.

4. Utilisation selon l'une quelconque des revendications 1 à 3, où le médicament est une composition alimentaire pour animaux, un complément alimentaire ou un produit alimentaire formulé pour une consommation humaine.

5. Utilisation selon l'une quelconque des revendications 1 à 3, où les LCPUFA comprennent au moins l'un de l'acide arachidonique, l'acide éicosapentaénoïque, l'acide docosapentaénoïque ou l'acide docosahexaénoïque.

6. Utilisation selon l'une quelconque des revendications 1 à 3, où les NORC comprennent au moins l'un de la L-arginine ou de ses dérivés.

7. Utilisation selon la revendication 6, où les LCPUFA sont dans une quantité d'au moins environ 0,1 % à environ 13 % en poids de la formulation et la L-arginine dans une quantité d'au moins environ 0,1 % à environ 12 % en poids de la formulation.

8. Utilisation selon l'une quelconque des revendications 1 à 3, où l'animal est un animal de compagnie.

9. Utilisation selon la revendication 8, où l'animal de compagnie est un chat ou un chien.

10. Utilisation selon l'une quelconque des revendications 1 à 3, où l'animal est un être humain.

11. Composition comprenant un ou plusieurs LCPUFA et un ou plusieurs NORC pour une utilisation dans la réduction d'une lésion cérébrale induite par une ischémie chez un animal, où la composition est administrée à l'animal sur une base régulière.

12. Composition selon la revendication 11, où la composition est une composition alimentaire pour animaux, un complément alimentaire ou un produit alimentaire formulé pour une consommation humaine.

13. Composition selon la revendication 11, dans laquelle les LCPUFA comprennent au moins l'un de l'acide arachidonique, l'acide éicosapentaénoïque, l'acide docosapentaénoïque ou l'acide docosahexaénoïque.

14. Composition selon la revendication 11, dans laquelle les NORC comprennent au moins l'un de la L-arginine ou de ses dérivés.

15. Composition selon la revendication 14, dans laquelle les LCPUFA sont dans une quantité d'au moins environ 0,1 % à environ 13 % en poids de la formulation et la L-arginine dans une quantité d'au moins environ 0,1 % à environ 12 % en poids de la formulation.

16. Composition selon la revendication 11, où l'animal est un animal de compagnie.

17. Composition selon la revendication 16, où l'animal de compagnie est un chat ou un chien.

18. Composition selon la revendication 11, où l'animal est un être humain.

19. Composition comprenant un ou plusieurs LCPUFA et un ou plusieurs NORC pour une utilisation destinée à amplifier l'intégrité vasculaire et à réduire une lésion cérébrale dans l'éventualité d'un épisode ischémique dans le cerveau.

20. Composition comprenant un ou plusieurs LCPUFA et un ou plusieurs NORC pour une utilisation destinée à améliorer l'intégrité vasculaire et à réduire l'apoptose cellulaire à l'emplacement d'une ischémie chez un animal.

21. Composition selon la revendication 20, où l'ischémie survient dans un tissu choisi parmi le cerveau, le coeur et le rein.

22. Composition comprenant un ou plusieurs LCPUFA et un ou plusieurs NORC pour une utilisation destinée à réduire une lésion cérébrale induite par une ischémie chez un animal.
